(19) Europäisches Patentamt
European Patent Office
Office européen des brevets

(11) **EP 3 686 176 A1**

(12) **EUROPÄISCHE PATENTANMELDUNG**

(43) Veröffentlichungstag:
**29.07.2020 Patentblatt 2020/31**

(51) Int Cl.:
***C07C 4/16*** *(2006.01)* ***C07C 15/04*** *(2006.01)*

(21) Anmeldenummer: **19154038.4**

(22) Anmeldetag: **28.01.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Linde GmbH**
**82049 Pullach (DE)**

(72) Erfinder:
• **Zander, Hans-Jörg**
**81479 München (DE)**
• **Tota, Akos**
**81479 München (DE)**

(74) Vertreter: **Frommberger, Moritz**
**m patent group**
**Postfach 33 04 29**
**80064 München (DE)**

(54) **VERFAHREN UND ANLAGE ZUR HERSTELLUNG VON BENZOL DURCH HYDRODEALKYLIERUNG**

(57) Die Erfindung betrifft ein Verfahren (100) zur Herstellung einer oder mehrerer Zielverbindungen wie Benzol, bei dem ein Reaktionseinsatzstrom, der eine oder mehrere Ausgangsverbindungen wie Alkylbenzole enthält, in einem Reaktorsystem (10) mit einem ersten, rückvermischenden Reaktor (11) und einem zweiten, nicht rückvermischenden Reaktor (12) unter Erhalt der einen oder den mehreren Zielverbindungen einer Umsetzungsreaktion wie einer Hydrodealkylierung unterworfen wird, und bei dem ein dem ersten Reaktor (11) entnommener Produktstrom zumindest zum Teil dem zweiten Reaktor (12) zugeführt wird. Hierbei ist vorgesehen, dass ein erster Teilstrom des Reaktionseinsatzstroms einer Vorwärmung (1) unterworfen und dem ersten Reaktor (11) zugeführt wird und ein zweiter Teilstrom des Reaktionseinsatzstroms unter Umgehung der Vorwärmung (1) und des ersten Reaktors (11) dem zweiten Reaktor (12) zugeführt wird. Eine entsprechende Anlage ist ebenfalls Gegenstand der vorliegenden Erfindung.

Fig. 1

EP 3 686 176 A1

**Beschreibung**

Die Erfindung betrifft ein Verfahren und eine Anlage zur Herstellung einer Zielverbindung mittels einer Umsetzungsreaktion, insbesondere von Benzol durch Hydrodealkylierung, gemäß den Oberbegriffen der unabhängigen Patentansprüche.

Stand der Technik

Benzol kann durch Hydrodealkylierung (HDA) von aromatischen Verbindungen wie Toluol, Xylolen und anderen alkylierten Aromaten, beispielsweise auch alkylierten Biphenylen, hergestellt werden. Die genannten Ausgangsverbindungen werden typischerweise in Form einer sogenannten BTX-Fraktion gewonnen, die beispielsweise unter Verwendung von sogenanntem Pyrolysebenzin gebildet werden kann, das beim Dampfspalten anfällt. Alternative Quellen sind das Reformat bei der katalytischen Reformierung sowie das Hydrierbenzin bei der Carbonisierung von Kohle.

Pyrolysebenzin aus der Dampfspaltung umfasst typischerweise überwiegend oder ausschließlich Kohlenwasserstoffe mit fünf bis zehn Kohlenstoffatomen, davon überwiegend Aromaten. Die in geringerem Anteil enthaltenen Aliphaten sind überwiegend ungesättigt und umfassen einen hohen Anteil an Acetylenen und Dienen. Das Pyrolysebenzin ist daher instabil und kann aufgrund der Polymerisationsneigung der genannten Komponenten nicht gelagert werden. Es wird daher in mehreren Schritten weiterbehandelt, und zwar im sogenannten Benzinweg einer der Dampfspaltung nachgeschalteten Trennung.

Hierbei kann beispielsweise zunächst eine selektive Hydrierung erfolgen, um Acetylene, Diene und Styrole zu Olefinen umzusetzen. Nach Abtrennung von höhermolekularen Komponenten kann das so behandelte Pyrolysebenzin einer Trennung zugeführt werden, in der eine Fraktion gebildet wird, die überwiegend oder ausschließlich Kohlenwasserstoffe mit sechs bis acht oder, soweit nach der vorigen Abtrennung verblieben, bis neun, Kohlenstoffatomen enthält. Es handelt sich hierbei um den sogenannten "Herzschnitt" (engl. Heart Cut).

Der Herzschnitt kann einer Hydrodesulfurierung unterworfen. Hier gebildeter Schwefelwasserstoff kann in einem Stripper ausgetrieben werden. Der entsprechend behandelte Herzschnitt kann anschließend einer Aromatenextraktion unterworfen werden, in welcher die BTX-Fraktion von den Aliphaten abgetrennt wird. Auch andere Verfahrensvarianten sind bekannt, die sich insbesondere in der Abfolge der jeweiligen Trennschritte unterscheiden können.

Die Ausgangsverbindungen für eine Hydrodealkylierung zur Herstellung von Benzol können grundsätzlich auch auf andere Weise gewonnen werden. Insbesondere ist die vorliegende Erfindung nicht auf die Gewinnung dieser Ausgangsverbindungen über den soeben geschilderten Weg beschränkt.

Bei der Hydrodealkylierung werden die Alkylreste i.d.R. unter Verbrauch jeweils eines Wasserstoffmoleküls und unter Bildung der entsprechenden Alkane vom Benzolring abgespalten. Im Falle von Biphenylen erfolgt in analoger Weise ferner eine Spaltung der Bindung zwischen den beiden Benzolringen. Es sind katalytische und thermische Hydrodealkylierungsverfahren bekannt. Zu weiteren Details bezüglich der Hydrodealkylierung sei auf Fachliteratur verwiesen, beispielsweise den Artikel "Benzene" in Ullmann's Encyclopedia of Industrial Chemistry, online seit 15. Juni 2000, DOI 10.1002/14356007.a03_475, insbesondere Abschnitt 5.3.1, "Hydrodealkylation".

Wie nachfolgend noch im Detail im Zusammenhang mit den erfindungsgemäß vorgeschlagenen Maßnahmen erläutert, wird eine Hydrodealkylierung typischerweise unter Verwendung von zweistufigen Reaktorsystemen durchgeführt. Die vorliegende Erfindung ist dabei aber nicht auf Verfahren zur Herstellung von Benzol durch Hydrodealkylierung von Alkylbenzolen beschränkt, wie unten noch erläutert, sondern eignet sich grundsätzlich zum Einsatz bei einer Vielzahl an Verfahren, bei denen entsprechende zweistufige Reaktorsysteme verwendet werden.

Die Erfindung stellt sich die Aufgabe, Verfahren, in denen zweistufige Reaktorsysteme der nachfolgend erläuterten Art eingesetzt werden, insbesondere ein Verfahren zur Herstellung von Benzol durch Hydrodealkylierung, in ihrer Leistung zu erhöhen.

Offenbarung der Erfindung

Vor diesem Hintergrund schlägt die vorliegende Erfindung ein Verfahren und eine Anlage zur Herstellung einer Zielverbindung mittels einer Umsetzungsreaktion, insbesondere von Benzol durch eine Hydrodealkylierung, mit den Merkmalen der unabhängigen Patentansprüche vor. Ausgestaltungen sind Gegenstand der abhängigen Patentansprüche sowie der nachfolgenden Beschreibung.

Die Hydrodealkylierung von Alkylbenzolen zur Herstellung von Benzol wird vorteilhafterweise unter adiabaten Bedingungen durchgeführt, d.h. in einem ungekühlten Reaktor, weil bei den vergleichsweise harschen Reaktionsbedingungen Flächen zum Wärmeaustausch ungünstig, weil unter anderem korrosionsanfällig, sind. Die bei entsprechenden Verfahren verwendeten Reaktoren sind typischerweise ausgemauert, so dass eine Anbringung entsprechender Wärmeaustauschflächen schwierig ist. Der Einsatz zur Hydrodealkylierung wird daher in herkömmlichen Verfahren lediglich vorgewärmt und in den Reaktor eingespeist, wobei die bei der Hydrodealkylierung freigesetzte Reaktionswärme die im Einsatz enthaltenen Edukte auf die erforderliche Reaktionstemperatur erwärmt.

Durch die gewünschte Austrittstemperatur aus dem Reaktor und die globale Wärmebilanz ist die maximale Temperatur, auf die der Einsatz vorgewärmt werden kann, jedoch begrenzt. Bei dieser maximalen Temperatur ist die Reaktion jedoch noch ausgesprochen langsam. Deshalb kann zur Hydrodealkylierung ein rückvermischender Reaktor eingesetzt werden, in dem der Einsatz mit dem heißen Reaktorinhalt vermischt wird und die Reaktion daher bei höherer Temperatur abläuft.

Rückvermischende und nicht rückvermischende Reaktoren sind aus dem Stand der Technik bekannt und in einschlägigen Lehrbüchern beschrieben. Wie beispielsweise bei J.F. Richardson und D.G. Peacock (Hrsg.), Coulson & Richardson's Chemical Engineering, Band 3, Chemical and Biochemical Reactors and Process Control, Butterworth Heinemann, 3. Auflage 1994, Abschnitt 1.10.3, "Reactor Type and Backmixing" oder bei J. Hagen, Chemiereaktoren, Auslegung und Simulation, Wiley-VCH, 2004, Abschnitt 2.1, "Betriebsweise und Grundtypen von Chemiereaktoren", erläutert, erfolgt in einem idealen Kolbenstromreaktor (Plug Flow Reaktor, PFR) keine Rückvermischung, wohingegen in einem einzelnen, kontinuierlich betriebenen Rührtankreaktor (Continuously Stirred Tank Reactor, CSTR) eine vollständige Rückvermischung vorliegt. Die vorliegende Erfindung ist selbstverständlich nicht auf die Verwendung "idealer", d.h. vollständig rückvermischender oder nicht rückvermischender Reaktoren beschränkt.

Der in einem entsprechenden Verfahren, und damit auch im Rahmen der vorliegenden Erfindung, eingesetzte rückvermischende Reaktor kann beispielsweise als der erwähnte Rührtankreaktor ausgebildet sein, der jedoch nicht notwendigerweise mechanisch gerührt werden muss. Allgemeiner gesprochen kann ein mittels geeigneter technischer Einrichtungen zwangsdurchmischter oder nicht zwangsdurchmischter, insbesondere ein gerührter oder nicht gerührter, Tankreaktor, der insbesondere kontinuierlich betrieben werden kann, verwendet werden. Wichtig ist die tatsächliche Rückvermischung in einem entsprechenden Reaktor bzw. die homogene Einmischung des Einsatzes in den erwärmten Reaktorinhalt. Unter einem "zwangsdurchmischten" Reaktor wird im hier verwendeten Sprachgebrauch ein Reaktor verstanden, der eine technische Mischeinrichtung wie ein Rührwerk oder eine Durchströmungseinrichtung zur Bereitstellung eines Blasenstroms, aufweist. Ein kontinuierlich betriebener Reaktor unterscheidet sich von einem nicht kontinuierlich betriebenen (Batch-)Reaktor dadurch, dass in diesen kontinuierlich Einsatz zugegeben und aus diesem kontinuierlich ein Produktgemisch abgezogen wird.

Der Nachteil eines rückvermischenden Reaktor gegenüber einem nicht rückvermischenden Reaktor wie beispielsweise einem Rohrreaktor bzw. Kolbenstromreaktor ist, dass bei hohen Umsätzen die Eduktkonzentration durch die Rückvermischung mit Produkt im gesamten Reaktor klein ist und die Reaktion entsprechend langsam abläuft. Deshalb kann die Hydrodealkylierungsreaktion zweistufig ausgeführt werden, wobei in einem ersten rückvermischenden Reaktor ein Teilumsatz erzielt und der Einsatz auf die zur Reaktion notwendige Temperatur angewärmt wird. In einem zweiten Reaktionsschritt in einem adiabaten Rohrreaktor werden bei dann abnehmender Eduktkonzentration der Umsatzgrad und die Temperatur weiter erhöht.

Beispielhafte Zahlenwerte für eine entsprechende zweistufige Reaktionsführung umfassen herkömmlicherweise eine Einsatztemperatur von 600 °C, eine Temperatur im ersten, rückvermischenden Reaktor sowie an dessen Austritt von 700 °C, und eine weitere Temperaturerhöhung im zweiten, nicht rückvermischenden Reaktor auf 720 °C.

Die maximale Temperatur in einem entsprechenden Reaktorsystem ist ebenfalls begrenzt, z.B. durch den thermischen Zerfall der Moleküle und die damit verbundene Verkokung oder durch materialtechnische Einschränkungen. Diese maximale Temperatur tritt am Ausgang der zweiten Reaktionsstufe auf. Entsprechend der adiabaten Reaktionsführung ist der Eintritt in die zweite Stufe kälter als der Austritt und damit auch die (mittlere) Reaktionstemperatur in der ersten Stufe. Die erste Stufe muss also kälter betrieben werden als es im Sinne einer maximalen Reaktionsgeschwindigkeit vorteilhaft wäre, um die maximal zulässige Temperatur am Reaktoraustritt nicht zu überschreiten. Dies verringert die Reaktionsgeschwindigkeit bzw. erfordert eine Vergrößerung des Reaktors.

Diese Probleme werden erfindungsgemäß dadurch gelöst, dass in einem Verfahren zur Herstellung einer Zielverbindung, bei dem ein Reaktionseinsatzstrom gebildet und einem Reaktorsystem mit einem ersten, rückvermischenden Reaktor und einem zweiten, nicht rückvermischenden Reaktor zugeführt wird, ein erster Teilstrom des Reaktionseinsatzstroms einer Vorwärmung unterworfen und dem ersten Reaktor zugeführt und ein zweiter Teilstrom des Reaktionseinsatzstroms unter Umgehung der Vorwärmung und des ersten Reaktors dem zweiten Reaktor zugeführt wird. Ein aus dem ersten Reaktor entnommener Produktstrom wird im Rahmen des Verfahrens zumindest zum Teil dem zweiten Reaktor zugeführt.

Die vorliegende Erfindung ist, wie erwähnt, nicht auf die Herstellung von Benzol als Zielverbindung und die Hydrodealkylierung von Alkylbenzolen beschränkt, sondern eignet prinzipiell für jede mehrstufige Reaktionsführung der erläuterten Art. Insbesondere eignet sich die vorliegende Erfindung für Fälle, in denen das Temperaturniveau der Reaktionsführung hoch ist, und außerdem insbesondere für Fälle, in denen unerwünschte Nebenreaktionen auftreten, deren Aktivierungsenergie niedriger ist als die der Hauptreaktionen. Die vorliegende Erfindung wird nachfolgend jedoch der Einfachheit halber unter Bezugnahme auf die Hydrodealkylierung und die Herstellung von Benzol als Zielverbindung beschrieben.

Die Erfindung schlägt, nochmals zusammengefasst, ein Verfahren zur Herstellung einer oder mehrerer Zielverbindungen vor, bei dem ein Reaktionseinsatzstrom, der eine oder mehrere Ausgangsverbindungen enthält, in einem Reaktorsystem mit einem ersten, rückvermischenden Reaktor und einem zweiten, nicht rückvermischenden Reaktor unter Erhalt der einen oder den mehreren Zielverbindungen einer Umsetzungsreaktion unterworfen wird, und bei dem ein dem ersten Reaktor entnommener Produktstrom zumindest zum Teil dem zweiten Reaktor zugeführt wird.

Es ist erfindungsgemäß vorgesehen, dass ein erster Teilstrom des Reaktionseinsatzstroms einer Vorwärmung unterworfen und dem ersten Reaktor zugeführt wird und ein zweiter Teilstrom des Reaktionseinsatzstroms unter Umgehung der Vorwärmung und des ersten Reaktors dem zweiten Reaktor zugeführt wird. Der zweite Teilstrom kann insbesondere zumindest teilweise mit dem ersten Produktstrom unter Erhalt einer Mischtemperatur zu einem Sammelstrom vermischt werden und der Sammelstrom kann dem zweiten Reaktor zugeführt werden.

Die eine oder die mehreren Ausgangsverbindungen umfassen in einem besonders bevorzugten Verfahren zur Herstellung von Benzol ein oder mehrere Alkylbenzole, die eine oder die mehreren Zielverbindungen umfassen in diesem Fall Benzol, und die Umsetzungsreaktion ist in diesem Fall eine Hydrodealkylierung.

Im Rahmen der vorliegenden Erfindung wird also ein, insbesondere vergleichsweise kleiner, erster Teilstrom (nachfolgend auch als Bypass bezeichnet) unter Verbleib des zweiten Teilstroms bereits vor der Vorwärmung von dem Reaktionseinsatzstrom abgezweigt, an der ersten Reaktionsstufe vorbeiführt, und dann wieder zugegeben.

Bezeichnet man mit $\varphi$ den Anteil des Bypasses am gesamtem Massenstrom des Reaktionseinsatzstroms, mit $T_0$ die Temperatur vor der Abzweigung, mit $T_1$ die Temperatur des Produktgemischs stromab des ersten Reaktors und mit $T_{mix}$ die Temperatur nach dem Zumischen des Bypasses, kann man die Wärmebilanz um den Mischungspunkt aufstellen (vereinfacht mit konstanten Stoffdaten):

$$\varphi\, m\, c_p\, T_0 + (1 - \varphi)\, m\, c_p\, T_1 = m\, c_p\, T_{mix}$$

Daraus folgt

$$T_1 = (T_{mix} - \varphi\, T_0) / (1 - \varphi),$$

und damit

$$T_1 > T_{mix}.$$

Bei gegebener Temperatur $T_{mix}$ am Mischungspunkt (der durch die Maximaltemperatur der gesamten Anordnung und die Temperaturerhöhung der zweiten Stufe gegeben ist) und gegebener Temperatur $T_0$ vor der Vorwärmung kann man also durch einen Bypass von wenigen Prozent, der den Umsatz in dem ersten Reaktor nicht wesentlich beeinträchtigt, eine signifikant höhere Betriebstemperatur in dem ersten Reaktor erreicht werden, ohne dabei die Maximaltemperatur am Austritt des zweiten Reaktors in unzulässiger Weise zu überschreiten. Die höhere Temperatur im ersten Reaktor erhöht die Reaktionsgeschwindigkeit, was dessen Auslegung zugute kommt.

Eine Kühlung zwischen dem ersten und zweiten Reaktor würde im Prinzip den gleichen Effekt haben, erfordert jedoch einen Apparat und metallische Kühlflächen, die in dem typischerweise verwendeten ausgemauerten Apparat ungünstig sind.

Je höher die Temperatur der Reaktion ist, desto niedriger fällt der Bypass aus. Deshalb ist die Anordnung insbesondere für Hochtemperaturreaktionen geeignet, zum Beispiel der Hydrodealkylierung von alkylsubstituierten Aromaten zur Gewinnung von Benzol. Wie erwähnt, profitieren jedoch auch andere Reaktionen von den erfindungsgemäß vorgeschlagenen Maßnahmen.

Wie bereits zuvor erwähnt, kann im Rahmen der vorliegenden Erfindung der erste Reaktor als Tankreaktor ausgebildet ist sein, wobei der erste Teilstrom des Reaktionseinsatzstroms in einen Tankinhalt des Tankreaktors eingemischt wird. Wie ebenfalls erwähnt, kann der Tankreaktor zwangsdurchmischt oder nicht zwangsdurchmischt sein, wobei der der Tankreaktor beispielsweise als gerührter und/oder Blasensäulenreaktor ausgebildet sein kann. Der im Rahmen der vorliegenden Erfindung eingesetzte zweite Reaktor kann insbesondere als Rohr- oder Kolbenstromreaktor ausgebildet sein.

Wie bereits erwähnt, kann der zweite Teilstrom des Reaktionseinsatzstroms in einem kleineren Mengenstrom bereitgestellt wird als der erste Teilstrom des Reaktionseinsatzstroms. Insbesondere kann der zweite Teilstrom weniger als 0,5%, 1 %, 2%, 3%, 4%, 5%, 10% oder 20% des (gesamten) Reaktionseinsatzstroms in das Reaktorsystem und der erste Teilstrom den verbleibenden Rest umfassen. Es ist grundsätzlich auch möglich und in bestimmten Fällen vorteilhaft, einen Mengenstrom des ersten und/oder des zweiten Reaktionseinsatzstroms in Abhängigkeit von einer Zieltemperatur in dem ersten und/oder dem zweiten Reaktor einzustellen. Auf diese Weise kann eine flexible Anpassung an die gewünschten Reaktionstemperaturen erfolgen und ggf. auf sich ändernde Bedingungen reagiert werden.

Im Rahmen der vorliegenden Erfindung erfolgt die Vorwärmung (des ersten Teilstroms) auf ein erstes Temperaturniveau, wobei das erste Temperaturniveau insbesondere bei 400 bis 800 °C, insbesondere bei 500 bis 700 °C lie gt. Der erste Reaktor wird im Rahmen der vorliegenden Erfindung vorteilhafterweise auf einem zweiten Temperaturniveau betrieben, wobei das zweite Temperaturniveau ebenfalls bei 400 bis 800 °C, aber dabei oberhalb des ersten Temperaturni veaus der Vorwärmung liegt. Es handelt sich dabei insbesondere um ein Temperaturniveau am Reaktoraustritt des ersten Reaktors. Der der zweite Reaktor wird vorteilhafterweise auf einem dritten Temperaturniveau betrieben, wobei das dritte Temperaturniveau ebenfalls bei 400 bis 800 °C und wiederum oberhalb des ersten, aber auch oberhalb des zweiten Temperaturniveaus liegt. Wiederum handelt es sich insbesondere um ein Temperaturniveau am Reaktoraustritt des zweiten Reaktors.

Schließlich erstreckt sich die vorliegende Erfindung, wie erwähnt, auch auf eine Anlage zur Herstellung einer oder mehrerer Zielverbindungen, wobei die Anlage dafür eingerichtet ist, einen Reaktionseinsatzstrom, der eine oder mehrere Ausgangsverbindungen enthält, in einem Reaktorsystem mit einem ersten, rückvermischenden Reaktor und einem zweiten, nicht rückvermischenden Reaktor unter Erhalt der einen oder den mehreren Zielverbindungen einer Umsetzungsreaktion zu unterwerfen und einen dem ersten Reaktor entnommenen Produktstrom zumindest zum Teil dem zweiten Reaktor zuzuführen.

Erfindungsgemäß zeichnet sich eine entsprechende Anlage durch Mittel aus, die dafür eingerichtet sind, einen ersten Teilstrom des Reaktionseinsatzstroms einer Vorwärmung zu unterwerfen und dem ersten Reaktor zuzuführen und einen zweiten Teilstrom des Reaktionseinsatzstroms unter Umgehung der Vorwärmung und des ersten Reaktors dem zweiten Reaktor zuzuführen.

Zu Merkmalen und Vorteilen einer entsprechenden Anlage, die insbesondere zur Durchführung eines Verfahrens eingerichtet ist, wie es zuvor erläutert wurde, sei auf die obigen, das erfindungsgemäß vorgeschlagene Verfahren und seine Varianten betreffenden Erläuterungen ausdrücklich verwiesen. Diese werden hier lediglich aus Gründen der Übersichtlichkeit nicht wiederholt.

Die vorliegende Erfindung wird nachfolgend unter Bezugnahme auf die beigefügte Zeichnung näher erläutert, die eine Ausgestaltung der Erfindung veranschaulicht.

Figurenbeschreibung

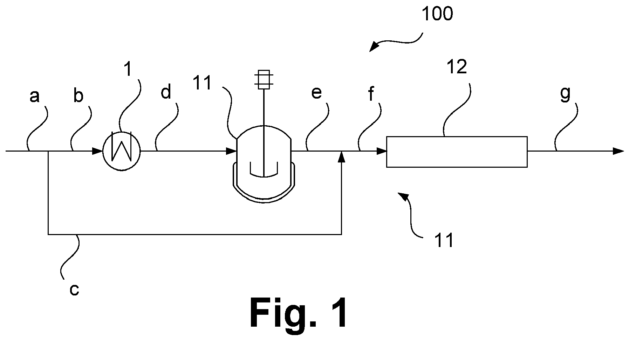

In Figur 1 ist ein Verfahren gemäß einer Ausführungsform der Erfindung anhand der verwendeten Komponenten in Form eines schematischen Ablaufplans veranschaulicht und insgesamt mit 100 bezeichnet. Die nachfolgend das Verfahren 100 betreffenden Erläuterungen gelten für eine entsprechende Anlage in gleicher Weise.

In dem Verfahren 100 wird im dargestellten Beispiel ein Reaktionseinsatzstrom a, der eine oder mehrere Ausgangsverbindungen enthält, in einem insgesamt mit 10 bezeichneten Reaktorsystem 10, das einen ersten, rückvermischenden Reaktor 11 und einen zweiten, nicht rückvermischenden Reaktor 12 aufweist, unter Erhalt der einen oder den mehreren Zielverbindungen einer Umsetzungsreaktion unterworfen. Wenngleich der erste Reaktor 11 hier als gerührter Tankreaktor veranschaulicht ist, muss im Rahmen der Erfindung kein Rührwerk verwendet werden. Ein dem ersten Reaktor 11 entnommener Produktstrom e wird in der hier veranschaulichten Ausgestaltung zumindest zum Teil dem zweiten Reaktor 12 zugeführt.

Im dargestellten Beispiel wird ein erster Teilstrom b des Reaktionseinsatzstroms a einer Vorwärmung in einem Wärmetauscher 1 unterworfen und dem ersten Reaktor 11 zugeführt. Ein zweiter Teilstrom c des Reaktionseinsatzstroms a wird dagegen unter Umgehung der Vorwärmung in dem Wärmetauscher 1 und unter Umgehung des ersten Reaktors 11 dem zweiten Reaktor 12 zugeführt. Der zweite Teilstrom c wird im dargestellten Beispiel zumindest teilweise mit dem ersten Produktstrom e unter Erhalt einer Mischtemperatur zu einem Sammelstrom f vermischt und der Sammelstrom f wird dem zweiten Reaktor 12 zugeführt.

Ein dem zweiten Reaktor 12 entnommener Produktstrom ist mit g bezeichnet. Dieser kann beispielsweise einer Trennung oder Aufbereitung bekannter Art zugeführt werden. Entsprechend kann auch die Bereitstellung des Reaktionseinsatzstroms a in an sich aus dem Stand der Technik bekannter Weise erfolgen.

**Patentansprüche**

1. Verfahren (100) zur Herstellung einer oder mehrerer Zielverbindungen, bei dem ein Reaktionseinsatzstrom, der eine oder mehrere Ausgangsverbindungen enthält, in einem Reaktorsystem (10) mit einem ersten, rückvermischenden Reaktor (11) und einem zweiten, nicht rückvermischenden Reaktor (12) unter Erhalt der einen oder den mehreren Zielverbindungen einer Umsetzungsreaktion unterworfen wird, und bei dem ein dem ersten Reaktor (11) entnommener Produktstrom zumindest zum Teil dem zweiten Reaktor (12) zugeführt wird, **dadurch gekennzeichnet, dass** ein erster Teilstrom des Reaktionseinsatzstroms einer Vorwärmung (1) unterworfen und dem ersten Reaktor (11) zugeführt wird und ein zweiter Teilstrom des Reaktionseinsatzstroms unter Umgehung der Vorwärmung (1) und des ersten Reaktors (11) dem zweiten Reaktor (12) zugeführt wird.

2. Verfahren (100) nach Anspruch 1, bei dem die eine oder die mehreren Ausgangsverbindungen ein oder mehrere Alkylbenzole umfassen, bei dem die eine oder die mehreren Zielverbindungen Benzol umfassen, und bei dem die Umsetzungsreaktion eine Hydrodealkylierung ist.

3. Verfahren (100) nach Anspruch 1 oder 2, bei dem der erste Reaktor (11) als Tankreaktor ausgebildet ist, wobei der erste Teilstrom des Reaktionseinsatzstroms in einen Tankinhalt des Tankreaktors eingemischt wird.

**4.** Verfahren (100) nach Anspruch 3, bei dem der Tankreaktor als gerührter und/oder Blasensäulenreaktor ausgebildet ist.

**5.** Verfahren (100) nach einem der vorstehenden Ansprüche, bei dem der zweite Reaktor (12) als Kolbenstromreaktor ausgebildet ist.

**6.** Verfahren (100) nach einem der vorstehenden Ansprüche, bei dem der zweite Teilstrom des Reaktionseinsatzstroms in einem kleineren Mengenstrom bereitgestellt wird als der erste Teilstrom des Reaktionseinsatzstroms.

**7.** Verfahren (100) nach einem der vorstehenden Ansprüche, bei dem ein Mengenstrom des ersten und/oder des zweiten Reaktionseinsatzstroms in Abhängigkeit von einer Zieltemperatur in dem ersten und/oder dem zweiten Reaktor (11, 12) eingestellt wird.

**8.** Verfahren (100) nach einem der vorstehenden Ansprüche, bei dem die Vorwärmung auf ein erstes Temperaturniveau erfolgt, wobei das erste Temperaturniveau bei 400 bis 800 °C liegt.

**9.** Verfahren (100) nach Anspruch 8, bei dem der erste Reaktor (11) auf einem zweiten Temperaturniveau betrieben wird, wobei das zweite Temperaturniveau bei 400 bis 800 °C und oberhalb des ersten Temperaturni veaus liegt.

**10.** Verfahren (100) nach Anspruch 9, bei dem der zweite Reaktor (11) auf einem dritten Temperaturniveau betrieben wird, wobei das dritte Temperaturniveau bei 400 bis 800 °C und oberhalb des ersten und zweiten Temperaturniveaus liegt.

**11.** Anlage zur Herstellung einer oder mehrerer Zielverbindungen, die dafür eingerichtet ist, einen Reaktionseinsatzstrom, der eine oder mehrere Ausgangsverbindungen enthält, in einem Reaktorsystem (10) mit einem ersten, rückvermischenden Reaktor (11) und einem zweiten, nicht rückvermischenden Reaktor (12) unter Erhalt der einen oder den mehreren Zielverbindungen einer Umsetzungsreaktion zu unterwerfen und einen dem ersten Reaktor (11) entnommenen Produktstrom zumindest zum Teil dem zweiten Reaktor (12) zuzuführen, **gekennzeichnet durch** Mittel, die dafür eingerichtet sind, einen ersten Teilstrom des Reaktionseinsatzstroms einer Vorwärmung (1) zu unterwerfen und dem ersten Reaktor (11) zuzuführen und einen zweiten Teilstrom des Reaktionseinsatzstroms unter Umgehung der Vorwärmung (1) und des ersten Reaktors (11) dem zweiten Reaktor (12) zuzuführen.

**12.** Anlage nach Anspruch 11, die zur Durchführung eines Verfahrens nach einem der Ansprüche 1 bis 10 eingerichtet ist.

**Fig. 1**

Europäisches
Patentamt

European
Patent Office

Office européen
des brevets

# EUROPÄISCHER RECHERCHENBERICHT

Nummer der Anmeldung

EP 19 15 4038

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WEISS, A.H. ET AL.: "Houdry Detol process shows good results at Crown Central refinery", OIL AND GAS JOURNAL, Bd. 60, Nr. 4, 1962, Seiten 64-66, XP009514467, ISSN: 0030-1388 * Seite 64 * * Seite 65; Abbildung * ----- | 1-12 | INV. C07C4/16 C07C15/04 |
| Y,D | Hillis O. Folkins: "Benzene" In: "Ullmann's Encyclopedia of Industrial Chemistry", 2012, Wiley-VCH, Weinheim, XP55603943, ISBN: 978-3-527-30673-2 Seiten 237-268, DOI: 10.1002/14356007.a03_475, * Seite 247, Absatz 5.3.1.; Abbildung 4 * ----- | 1-12 | |
| Y | US 2016/362351 A1 (NAGAKI DICK A [US] ET AL) 15. Dezember 2016 (2016-12-15) * Seite 24, Absatz [0221]; Abbildung 4E * ----- | 11,12 | |
| Y | WO 2010/104920 A1 (SHELL OIL COMPANY; SHELL INTERNATIONALE RESEARCH MAATSCHAPPIJ B.V.) 16. September 2010 (2010-09-16) * Seite 11, Zeile 29 - Seite 12, Zeile 24 * ----- | 1-12 | |
| A | US 3 340 318 A (CARR NORMAN L ET AL) 5. September 1967 (1967-09-05) * das ganze Dokument * ----- | 1-12 | |
| A | US 3 374 280 A (CARR NORMAN L ET AL) 19. März 1968 (1968-03-19) * das ganze Dokument * ----- | 1-12 | |

RECHERCHIERTE SACHGEBIETE (IPC)

C07C

-/--

Der vorliegende Recherchenbericht wurde für alle Patentansprüche erstellt

| Recherchenort | Abschlußdatum der Recherche | Prüfer |
|---|---|---|
| München | 18. Juli 2019 | Kiernan, Andrea |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

Seite 1 von 2

Europäisches
Patentamt

European
Patent Office

Office européen
des brevets

## EUROPÄISCHER RECHERCHENBERICHT

**Nummer der Anmeldung**

EP 19 15 4038

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 3 591 651 A (CARR NORMAN L ET AL) 6. Juli 1971 (1971-07-06) * das ganze Dokument * ----- | 1-12 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 18. Juli 2019 | Kiernan, Andrea |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes
    Dokument

EPO FORM 1503 03.82 (P04C03)

Seite 2 von 2

EP 3 686 176 A1

## ANHANG ZUM EUROPÄISCHEN RECHERCHENBERICHT
## ÜBER DIE EUROPÄISCHE PATENTANMELDUNG NR.

EP 19 15 4038

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-07-2019

| Im Recherchenbericht angeführtes Patentdokument | Datum der Veröffentlichung | Mitglied(er) der Patentfamilie | Datum der Veröffentlichung |
|---|---|---|---|
| US 2016362351 A1 | 15-12-2016 | CN 106536456 A<br>TW 201716358 A<br>US 2016362351 A1<br>WO 2016201256 A1 | 22-03-2017<br>16-05-2017<br>15-12-2016<br>15-12-2016 |
| WO 2010104920 A1 | 16-09-2010 | CN 102482179 A<br>EA 201171126 A1<br>SG 174856 A1<br>SG 10201400509R A<br>US 2012029256 A1<br>WO 2010104920 A1 | 30-05-2012<br>30-04-2012<br>28-11-2011<br>30-10-2014<br>02-02-2012<br>16-09-2010 |
| US 3340318 A | 05-09-1967 | DE 1668154 A1<br>ES 347406 A1<br>FR 1553004 A<br>GB 1196960 A<br>NL 6715865 A<br>US 3340318 A | 25-03-1971<br>01-06-1969<br>10-01-1969<br>01-07-1970<br>24-05-1968<br>05-09-1967 |
| US 3374280 A | 19-03-1968 | DE 1618477 A1<br>ES 342000 A1<br>GB 1175849 A<br>NL 6708582 A<br>US 3374280 A | 22-10-1970<br>16-07-1968<br>23-12-1969<br>21-12-1967<br>19-03-1968 |
| US 3591651 A | 06-07-1971 | ES 376755 A2<br>FR 2034929 A2<br>GB 1292701 A<br>JP S518929 B1<br>US 3591651 A | 01-06-1972<br>18-12-1970<br>11-10-1972<br>22-03-1976<br>06-07-1971 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- Benzene. Ullmann's Encyclopedia of Industrial Chemistry. Juni 2000, 15
- Chemical and Biochemical Reactors and Process Control. Coulson & Richardson's Chemical Engineering. Butterworth Heinemann, 1994, vol. 3
- Reactor Type and Backmixing. **J. HAGEN.** Chemiereaktoren, Auslegung und Simulation. Wiley-VCH, 2004